(19) Europäisches Patentamt
European Patent Office
Office européen des brevets

(11) **EP 3 473 645 A1**

(12) **EUROPEAN PATENT APPLICATION**

(43) Date of publication:
**24.04.2019 Bulletin 2019/17**

(51) Int Cl.:
**C07K 14/415** (2006.01)   **C12N 15/82** (2006.01)

(21) Application number: **17197076.7**

(22) Date of filing: **18.10.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
• **SESVanderHave N.V.**
**3300 Tienen (BE)**

• **Michigan Technological University**
**Houghton, Michigan 49931-1295 (US)**

(72) Inventor: **Busov, Victor**
**Houghton, MI Michigan 49931 (US)**

(74) Representative: **de Diesbach, Philippe**
**SESVanderHave NV**
**Soldatenplein Z2**
**3300 Tienen (BE)**

(54) **GENE FOR YIELD INCREASE**

(57)   A DNA vector suitable for the expression in an host cell of SEQ.ID.NO:2, the corresponding host cell and transgenic plant, and a method for the identification or the generation of plants having increased nitrogen use efficiency.

Figure 2

SEQUENCE LISTING

<110> SES VanderHave

<120> GENE FOR YIELD INCREASE

<130> NuePop

<160> 14

<170> PatentIn version 3.5

<210> 1
<211> 1059
<212> DNA
<213> Populus alba


<220>
<221> CDS
<222> (1)..(1059)

<400> 1

```
atg gag ggc acc aat tcg tcg tcc cgg tca cag cac ccg cag ctg cca      48
Met Glu Gly Thr Asn Ser Ser Ser Arg Ser Gln His Pro Gln Leu Pro
1               5                   10                  15

ccc ggg ttc cgg ttc cac cct act gac gaa gag cta gtg gtc cac tac      96
Pro Gly Phe Arg Phe His Pro Thr Asp Glu Glu Leu Val Val His Tyr
                20                  25                  30

cta aag aag aag gcc gca tcg gtt ccg cta ccc gtt aca att ata gct     144
Leu Lys Lys Lys Ala Ala Ser Val Pro Leu Pro Val Thr Ile Ile Ala
            35                  40                  45

gag att gat cta tac aaa ttt ggt cca tgg gag ctg cca agt aag gct     192
Glu Ile Asp Leu Tyr Lys Phe Gly Pro Trp Glu Leu Pro Ser Lys Ala
        50                  55                  60

act ttt gga gag caa gag tgg tac ttt ttc agt cct aga gac agg aaa     240
Thr Phe Gly Glu Gln Glu Trp Tyr Phe Phe Ser Pro Arg Asp Arg Lys
65                  70                  75                  80

tac ccc aac ggt gct agg cct aac aga gct gca act tca ggg tat tgg     288
Tyr Pro Asn Gly Ala Arg Pro Asn Arg Ala Ala Thr Ser Gly Tyr Trp
                85                  90                  95

aaa gct acc ggg act gat aag cct ata tta aca tct aat gga gct caa     336
Lys Ala Thr Gly Thr Asp Lys Pro Ile Leu Thr Ser Asn Gly Ala Gln
                100                 105                 110

aag gtt ggt gtc aaa aag gcg ctg gtt ttt tat ggt ggg aag cca ccg     384
Lys Val Gly Val Lys Lys Ala Leu Val Phe Tyr Gly Gly Lys Pro Pro
            115                 120                 125

aag ggg att aaa acc gat tgg att atg cat gaa tat cgc ctt atc gaa     432
Lys Gly Ile Lys Thr Asp Trp Ile Met His Glu Tyr Arg Leu Ile Glu
        130                 135                 140

aac agt tct agc cca agg ccc cct gct gct gat tct gcc acc aag aaa     480
Asn Ser Ser Ser Pro Arg Pro Pro Ala Ala Asp Ser Ala Thr Lys Lys
145                 150                 155                 160
```

```
ggc ggc tct tta agg ctt gat gat tgg gtt ctc tgc cgg att tac aag          528
Gly Gly Ser Leu Arg Leu Asp Asp Trp Val Leu Cys Arg Ile Tyr Lys
                165                 170                 175

aaa aac aac tct cag aga ccg atg gat caa agg gac aag gag gat tca          576
Lys Asn Asn Ser Gln Arg Pro Met Asp Gln Arg Asp Lys Glu Asp Ser
                180                 185                 190

atg gag ggc atg ttt gct aca ctg caa aat tcc ggc cac caa aat ccg          624
Met Glu Gly Met Phe Ala Thr Leu Gln Asn Ser Gly His Gln Asn Pro
                195                 200                 205

aaa ccc cct tca gct tca aag cac aca act tat gca tct tta ctt gtg          672
Lys Pro Pro Ser Ala Ser Lys His Thr Thr Tyr Ala Ser Leu Leu Val
                210                 215                 220

aat gaa gat acc ttc ttt gaa ggg ata cta aca gga gat ggc atg caa          720
Asn Glu Asp Thr Phe Phe Glu Gly Ile Leu Thr Gly Asp Gly Met Gln
225                 230                 235                 240

aat ggt tcc att tct caa tta cca tct tca agc tca aag cca aac atg          768
Asn Gly Ser Ile Ser Gln Leu Pro Ser Ser Ser Ser Lys Pro Asn Met
                245                 250                 255

tcc atg gcc gcc cca gtc tcc aca aat act ttt acg gcg aca cgc gcc          816
Ser Met Ala Ala Pro Val Ser Thr Asn Thr Phe Thr Ala Thr Arg Ala
                260                 265                 270

ctc cct cct cat cag tac tgg aac gac gct aca gga tca cca gtg ggg          864
Leu Pro Pro His Gln Tyr Trp Asn Asp Ala Thr Gly Ser Pro Val Gly
                275                 280                 285

ttg gct aat tca tca ggg aag cgc ttt cac ggt gaa ctt aat agt ggt          912
Leu Ala Asn Ser Ser Gly Lys Arg Phe His Gly Glu Leu Asn Ser Gly
                290                 295                 300

att acc gga act caa gag gat aac acc tcc ttt gtt tct atg ctc aac          960
Ile Thr Gly Thr Gln Glu Asp Asn Thr Ser Phe Val Ser Met Leu Asn
305                 310                 315                 320

cag ctt cca cag agc aca cca atg gtc cac cca agt act ctt ctt gat          1008
Gln Leu Pro Gln Ser Thr Pro Met Val His Pro Ser Thr Leu Leu Asp
                325                 330                 335

ggt gtt ttg aga caa ccg ttt caa ctt tct agc ttg aat tgg aac tca          1056
Gly Val Leu Arg Gln Pro Phe Gln Leu Ser Ser Leu Asn Trp Asn Ser
                340                 345                 350

taa                                                                       1059
```

```
<210>  2
<211>  352
<212>  PRT
<213>  Populus alba

<400>  2

Met Glu Gly Thr Asn Ser Ser Ser Arg Ser Gln His Pro Gln Leu Pro
1               5                   10                  15


Pro Gly Phe Arg Phe His Pro Thr Asp Glu Glu Leu Val Val His Tyr
```

|  | 20 |  |  |  | 25 |  |  |  |  | 30 |  |  |  |
|---|---|---|---|---|---|---|---|---|---|---|---|---|---|

Leu Lys Lys Lys Ala Ala Ser Val Pro Leu Pro Val Thr Ile Ile Ala
35                  40              45

Glu Ile Asp Leu Tyr Lys Phe Gly Pro Trp Glu Leu Pro Ser Lys Ala
50              55              60

Thr Phe Gly Glu Gln Glu Trp Tyr Phe Phe Ser Pro Arg Asp Arg Lys
65          70              75                  80

Tyr Pro Asn Gly Ala Arg Pro Asn Arg Ala Ala Thr Ser Gly Tyr Trp
85              90              95

Lys Ala Thr Gly Thr Asp Lys Pro Ile Leu Thr Ser Asn Gly Ala Gln
100              105              110

Lys Val Gly Val Lys Lys Ala Leu Val Phe Tyr Gly Gly Lys Pro Pro
115              120              125

Lys Gly Ile Lys Thr Asp Trp Ile Met His Glu Tyr Arg Leu Ile Glu
130              135              140

Asn Ser Ser Ser Pro Arg Pro Pro Ala Ala Asp Ser Ala Thr Lys Lys
145              150              155              160

Gly Gly Ser Leu Arg Leu Asp Asp Trp Val Leu Cys Arg Ile Tyr Lys
165              170              175

Lys Asn Asn Ser Gln Arg Pro Met Asp Gln Arg Asp Lys Glu Asp Ser
180              185              190

Met Glu Gly Met Phe Ala Thr Leu Gln Asn Ser Gly His Gln Asn Pro
195              200              205

Lys Pro Pro Ser Ala Ser Lys His Thr Thr Tyr Ala Ser Leu Leu Val
210              215              220

Asn Glu Asp Thr Phe Phe Glu Gly Ile Leu Thr Gly Asp Gly Met Gln
225              230              235              240

Asn Gly Ser Ile Ser Gln Leu Pro Ser Ser Ser Lys Pro Asn Met
245              250              255

Ser Met Ala Ala Pro Val Ser Thr Asn Thr Phe Thr Ala Thr Arg Ala
260              265              270

```
Leu Pro Pro His Gln Tyr Trp Asn Asp Ala Thr Gly Ser Pro Val Gly
        275                 280                 285


Leu Ala Asn Ser Ser Gly Lys Arg Phe His Gly Glu Leu Asn Ser Gly
        290                 295                 300


Ile Thr Gly Thr Gln Glu Asp Asn Thr Ser Phe Val Ser Met Leu Asn
305                 310                 315                 320


Gln Leu Pro Gln Ser Thr Pro Met Val His Pro Ser Thr Leu Leu Asp
                325                 330                 335


Gly Val Leu Arg Gln Pro Phe Gln Leu Ser Ser Leu Asn Trp Asn Ser
                340                 345                 350
```

```
<210>  3
<211>  1320
<212>  DNA
<213>  Beta vulgaris


<220>
<221>  CDS
<222>  (56)..(1081)


<400>  3
tatcataata aagttttaac acacaatcaa actcataaaa aatattacaa aagct atg      58
                                                                Met
                                                                1


gaa agc aca gac tca tct tcc acc caa cct tac cct cag ctt cct cct     106
Glu Ser Thr Asp Ser Ser Ser Thr Gln Pro Tyr Pro Gln Leu Pro Pro
            5                   10                  15


gga ttt cgc ttc cat cct act gat gaa gaa ctt att gtt cat tac ctt     154
Gly Phe Arg Phe His Pro Thr Asp Glu Glu Leu Ile Val His Tyr Leu
        20                  25                  30


aaa agg aag gcc tct tct tcc cct ctt cct gtt tcg att atc gcc gag     202
Lys Arg Lys Ala Ser Ser Ser Pro Leu Pro Val Ser Ile Ile Ala Glu
    35                  40                  45


gtc gat ttg tac aag ttt gat cca tgg gag ctt cca agt aag gca atc     250
Val Asp Leu Tyr Lys Phe Asp Pro Trp Glu Leu Pro Ser Lys Ala Ile
50                  55                  60                  65


ttt gga gaa cag gag tgg tac ttt ttt agt cct aga gac cga aag tac     298
Phe Gly Glu Gln Glu Trp Tyr Phe Phe Ser Pro Arg Asp Arg Lys Tyr
                70                  75                  80


cca aat ggg gcc cgg cct aat aga gct gca act tca ggt tat tgg aaa     346
Pro Asn Gly Ala Arg Pro Asn Arg Ala Ala Thr Ser Gly Tyr Trp Lys
        85                  90                  95


gct act gga act gat aag cca ata ttg aca tct aat gga aat caa aaa     394
Ala Thr Gly Thr Asp Lys Pro Ile Leu Thr Ser Asn Gly Asn Gln Lys
        100                 105                 110
```

14

```
gtt ggt gtt aag aaa gct ctt gtt ttc tat ggt ggt aaa cct cct aga          442
Val Gly Val Lys Lys Ala Leu Val Phe Tyr Gly Gly Lys Pro Pro Arg
        115             120             125

gga atc aaa act gat tgg att atg cac gaa tat cgc att att gac gcc          490
Gly Ile Lys Thr Asp Trp Ile Met His Glu Tyr Arg Ile Ile Asp Ala
130             135             140             145

aat tct acc tcc aaa ttg cct cat cct ctc gtc gat cat cct tca aag          538
Asn Ser Thr Ser Lys Leu Pro His Pro Leu Val Asp His Pro Ser Lys
                150             155             160

aaa gca tct ctt cgg ctc gat gat tgg gtt tta tgc cgg att tac aag          586
Lys Ala Ser Leu Arg Leu Asp Asp Trp Val Leu Cys Arg Ile Tyr Lys
            165             170             175

aag aac aac act cag aga acg atg gac cac gaa aga gat gat cta att          634
Lys Asn Asn Thr Gln Arg Thr Met Asp His Glu Arg Asp Asp Leu Ile
        180             185             190

gag gag atg ata aag cat caa aag ttg atg aga aca att tgt cca aaa          682
Glu Glu Met Ile Lys His Gln Lys Leu Met Arg Thr Ile Cys Pro Lys
        195             200             205

aat aca act tct atg atg gat cat aac aat aat gat caa aat tta ttt          730
Asn Thr Thr Ser Met Met Asp His Asn Asn Asn Asp Gln Asn Leu Phe
210             215             220             225

gat ctt tca caa tta ggc act tca act tca agg cat gat ttg tat gac          778
Asp Leu Ser Gln Leu Gly Thr Ser Thr Ser Arg His Asp Leu Tyr Asp
            230             235             240

atg caa gga aat gat cac aag cgc gaa tta cca gtt tct ctg tat tgg          826
Met Gln Gly Asn Asp His Lys Arg Glu Leu Pro Val Ser Leu Tyr Trp
            245             250             255

gat gat caa atg ggt tcc att gga tca tca tct gga agg cga ttt cat          874
Asp Asp Gln Met Gly Ser Ile Gly Ser Ser Ser Gly Arg Arg Phe His
            260             265             270

cat cca caa tcg gat cag aca act ggg agt act ggt act gat gga aat          922
His Pro Gln Ser Asp Gln Thr Thr Gly Ser Thr Gly Thr Asp Gly Asn
            275             280             285

agt tca tca ttt gtt tcc atg tta agt caa ctt cct cag ggg atc aat          970
Ser Ser Ser Phe Val Ser Met Leu Ser Gln Leu Pro Gln Gly Ile Asn
290             295             300             305

aat act act act gtc cct ttt agc atg ggg agt ccc ttg tta gtt ggg         1018
Asn Thr Thr Thr Val Pro Phe Ser Met Gly Ser Pro Leu Leu Val Gly
            310             315             320

aac att ggg gat gga gtt tta agg cca ccc ttt cag ctt tct ggt ggg         1066
Asn Ile Gly Asp Gly Val Leu Arg Pro Pro Phe Gln Leu Ser Gly Gly
            325             330             335

aca tgg aat tca tag tttcttcgat catcaaattc atcatcatga tatatatata        1121
Thr Trp Asn Ser
            340

tatatacttt aattttatgt atacatattg aagcaaatac agtgaaaaga aactatatac       1181

atttgttaag tgaataccaa gtgaacttta tagtatattg tgagcatttt tcatggaaag       1241
```

```
ctagactatt ttgtggtaag ttcagttctc atccccttaa gtactcttat aatcatggta    1301

tattgagatt gttatcata                                                  1320
```

<210> 4
<211> 341
<212> PRT
<213> Beta vulgaris

<400> 4

```
Met Glu Ser Thr Asp Ser Ser Ser Thr Gln Pro Tyr Pro Gln Leu Pro
1               5                   10                  15


Pro Gly Phe Arg Phe His Pro Thr Asp Glu Glu Leu Ile Val His Tyr
            20                  25                  30


Leu Lys Arg Lys Ala Ser Ser Ser Pro Leu Pro Val Ser Ile Ile Ala
        35                  40                  45


Glu Val Asp Leu Tyr Lys Phe Asp Pro Trp Glu Leu Pro Ser Lys Ala
    50                  55                  60


Ile Phe Gly Glu Gln Glu Trp Tyr Phe Phe Ser Pro Arg Asp Arg Lys
65                  70                  75                  80


Tyr Pro Asn Gly Ala Arg Pro Asn Arg Ala Ala Thr Ser Gly Tyr Trp
                85                  90                  95


Lys Ala Thr Gly Thr Asp Lys Pro Ile Leu Thr Ser Asn Gly Asn Gln
            100                 105                 110


Lys Val Gly Val Lys Lys Ala Leu Val Phe Tyr Gly Gly Lys Pro Pro
        115                 120                 125


Arg Gly Ile Lys Thr Asp Trp Ile Met His Glu Tyr Arg Ile Ile Asp
    130                 135                 140


Ala Asn Ser Thr Ser Lys Leu Pro His Pro Leu Val Asp His Pro Ser
145                 150                 155                 160


Lys Lys Ala Ser Leu Arg Leu Asp Asp Trp Val Leu Cys Arg Ile Tyr
                165                 170                 175


Lys Lys Asn Asn Thr Gln Arg Thr Met Asp His Glu Arg Asp Asp Leu
            180                 185                 190


Ile Glu Glu Met Ile Lys His Gln Lys Leu Met Arg Thr Ile Cys Pro
            195                 200                 205
```

```
Lys Asn Thr Thr Ser Met Met Asp His Asn Asn Asn Asp Gln Asn Leu
    210             215             220

Phe Asp Leu Ser Gln Leu Gly Thr Ser Thr Ser Arg His Asp Leu Tyr
    225             230             235             240

Asp Met Gln Gly Asn Asp His Lys Arg Glu Leu Pro Val Ser Leu Tyr
                245             250             255

Trp Asp Asp Gln Met Gly Ser Ile Gly Ser Ser Ser Gly Arg Arg Phe
            260             265             270

His His Pro Gln Ser Asp Gln Thr Thr Gly Ser Thr Gly Thr Asp Gly
        275             280             285

Asn Ser Ser Ser Phe Val Ser Met Leu Ser Gln Leu Pro Gln Gly Ile
    290             295             300

Asn Asn Thr Thr Thr Val Pro Phe Ser Met Gly Ser Pro Leu Leu Val
305             310             315             320

Gly Asn Ile Gly Asp Gly Val Leu Arg Pro Pro Phe Gln Leu Ser Gly
            325             330             335

Gly Thr Trp Asn Ser
            340
```

```
<210>  5
<211>  362
<212>  PRT
<213>  Glycine max

<400>  5
```

```
Met Asp Ser Arg Asp Ser Ser Ser Gly Ser Gln His Pro His Leu Pro
1               5               10              15

Pro Gly Phe Arg Phe His Pro Thr Asp Glu Glu Leu Val Val His Tyr
            20              25              30

Leu Lys Arg Lys Ala Ala Ser Ala Pro Leu Pro Val Ala Ile Ile Ala
        35              40              45

Asp Val Asp Leu Tyr Lys Phe Asp Pro Trp Glu Leu Pro Ser Lys Ala
        50              55              60

Thr Phe Gly Glu Gln Glu Trp Tyr Phe Phe Ser Pro Arg Asp Arg Lys
65              70              75              80
```

```
Tyr Pro Asn Gly Ala Arg Pro Asn Arg Ala Ala Thr Ser Gly Tyr Trp
                85                  90                  95

Lys Ala Thr Gly Thr Asp Lys Pro Ile Leu Thr Thr Tyr Gly His His
            100             105             110

Lys Val Gly Val Lys Lys Ala Leu Val Phe Tyr Gly Gly Lys Pro Pro
        115             120             125

Lys Gly Val Lys Thr Asn Trp Ile Met His Glu Tyr Arg Leu Val Asp
        130             135             140

Asp Ser Phe Asn Ser Ser Ser Lys Pro Pro Pro Leu Val Pro His Asn
145             150             155                 160

Lys Lys Asn Ser Leu Arg Leu Asp Asp Trp Val Leu Cys Arg Ile Tyr
                165             170             175

Lys Lys Ser Asn Asn Thr Thr Leu Pro Arg Pro Pro Met Met Glu His
            180             185             190

Glu Glu Glu Leu Ser Met Asp Asn Met Leu Pro Thr Met Ser Thr Leu
            195             200             205

Ser Met Ala Asn Asn Lys Met Gln Asn Pro Lys Pro Pro Ser Arg Ser
        210             215             220

Thr Ser Tyr Gly Pro Leu Gly Leu Glu Asn Asp Asp Asn Phe Phe Glu
225             230             235             240

Gly Val Leu Ala Val Asp Gln Ser Met Gln Asn Gly Ser Asp Ser His
            245             250             255

Ile Phe Ser Pro Asn Ser Lys Gly Asp Asn Asn Asn Asn Asn Asn Asn
        260             265             270

Asn Ala Ser Asn Phe Pro Ile Lys Arg Ala Leu Ile Thr Ser Gln Leu
        275             280             285

Trp Asn Glu Thr Gly Ser Pro Gly Ser Ser Ser Cys Lys Arg Phe
    290             295             300

His Gly Asp Leu Asn Cys Gly Cys Ser Asn Ala Glu Asp Asn Asn Asp
305             310             315             320

Ser Phe Ile Ser Leu Leu Ser Gln Phe Pro Gln Asn Ala Thr Phe Gln
```

|  |  |  | 325 |  |  |  | 330 |  |  |  |  |  | 335 |  |
|---|---|---|---|---|---|---|---|---|---|---|---|---|---|---|

Pro Asn Ala Ile His Gly Ser Val Glu Asp Asp Gly Ala Pro Arg Gln
        340            345             350

Gln Phe His Leu His Gly Ile Asn Trp Asn
        355            360

<210>  6
<211>  321
<212>  PRT
<213>  Brassica napus

<400>  6

Met Glu Asn Met Gly Asp Ser Thr Ile Gly Pro Gly His Pro His Leu
1            5          10          15

Pro Pro Gly Phe Arg Phe His Pro Thr Asp Glu Glu Leu Val Val His
        20            25          30

Tyr Leu Lys Lys Lys Ala Ala Ser Ile Pro Leu Pro Val Ser Ile Ile
        35            40          45

Ala Glu Ile Asp Leu Tyr Lys Phe Asp Pro Trp Glu Leu Pro Ser Lys
      50            55          60

Ala Ser Phe Gly Glu Gln Glu Trp Tyr Phe Phe Ser Pro Arg Asp Arg
65            70          75          80

Lys Tyr Pro Asn Gly Val Arg Pro Asn Arg Ala Ala Thr Ser Gly Tyr
        85            90          95

Trp Lys Ala Thr Gly Thr Asp Lys Pro Ile Phe Thr Cys Asn Ser His
        100           105         110

Lys Val Gly Val Lys Lys Ala Leu Val Phe Tyr Gly Gly Lys Pro Pro
        115           120         125

Lys Gly Ile Lys Thr Asp Trp Ile Met His Glu Tyr Arg Leu Thr Asp
        130           135         140

Gly Asn Leu Asn Thr Ala Ala Lys Pro Pro Asp Ser Thr Thr Ser Arg
145            150          155         160

Lys Asn Ser Leu Arg Leu Asp Asp Trp Val Leu Cys Arg Ile Tyr Lys
        165           170         175

Lys Asn Ser Ser Gln Arg Pro Thr Met Glu Arg Val Leu Leu Arg Glu

                        180                        185                              190


    Asp Leu Met Glu Gly Met Leu Ser Lys Ser Ser Ala Asn Ser Ser Ser
            195                    200                    205


    Thr Ser Val Leu Asp Asn Asn Asn Asn Asn Asn Asn Glu Glu His Phe
            210                    215                    220


    Phe Asp Gly Met Ala Val Ser Ser Asp Lys Arg Ser Leu Cys Gly Gln
    225                    230                    235                    240


    Tyr Arg Ile Gly His Glu Ala Ser Gly Ser Ser Ser Phe Gly Ser Phe
                    245                    250                    255


    Leu Ser Ser Lys Arg Phe His His Thr Ser Asp Leu Asn Asn Asp Asn
            260                    265                    270


    Tyr Asn Val Ser Phe Val Ser Met Leu Ser Glu Ile Pro Gln Ser Ser
            275                    280                    285


    Gly Phe His Gly Asn Gly Val Ile Asp Thr Thr Ser Thr Leu Ala Asp
            290                    295                    300


    His Gly Val Leu Arg Gln Ala Phe Gln Leu Pro Asn Met Asn Trp His
    305                    310                    315                    320


    Pro



    <210>  7
    <211>  354
    <212>  PRT
    <213>  Gossypium hirsutum

    <400>  7

    Met Lys Asn Tyr Lys Glu Asn Val Lys Tyr Cys Lys Leu Met Glu Ser
    1                  5                  10                    15


    Thr Asp Pro Ser Ser Thr Ser Pro His Pro Gln Leu Pro Pro Gly Phe
                    20                    25                    30


    Arg Phe His Pro Thr Asp Glu Glu Leu Val Val His Tyr Leu Lys Arg
            35                    40                    45


    Lys Val Ala Ser Val Pro Leu Pro Val Thr Ile Ile Ala Glu Val Asp
            50                    55                    60


    Leu Tyr Lys Phe Asp Pro Trp Glu Leu Pro Asn Lys Ala Ser Phe Gly

```
          65                        70                        75                        80


          Glu Gln Glu Trp Tyr Phe Phe Ser Pro Arg Asp Arg Lys Tyr Pro Asn
                      85                  90                  95


          Gly Ala Arg Pro Asn Arg Ala Thr Thr Ser Gly Tyr Trp Lys Ala Thr
                     100                 105                 110


          Gly Thr Asp Lys Pro Ile Ile Thr Ser Asn Gly Asn Gln Lys Val Gly
                     115                 120                 125


          Val Lys Lys Ala Leu Val Phe Tyr Gly Gly Lys Pro Pro Lys Gly Ile
                     130                 135                 140


          Lys Thr Asn Trp Ile Met His Glu Tyr Arg Leu Ile Asp Ser Asn Ser
          145                 150                 155                 160


          Asn Ser Lys Ala Gln Ile Val Asp Leu Pro Asn Arg Arg Ala Ser Leu
                     165                 170                 175


          Arg Leu Asp Asp Trp Val Leu Cys Arg Ile Tyr Lys Lys Asn Asn Ser
                     180                 185                 190


          Gln Arg Leu Met Glu Lys Asp Lys Asn His Ser Ala Leu Pro Ser Phe
                     195                 200                 205


          Ser His Gln Asn His Gly Ser Leu Val Glu His Glu Glu Asn Cys Tyr
                     210                 215                 220


          Lys Arg Ile Leu Thr Gly Asp Ser Met Gln Asn Ser Ser Ile Ser Gln
          225                 230                 235                 240


          Thr Ala Val Ser Ser Ser Ser Lys Gln Ser Leu Pro Met Ala Phe Leu
                     245                 250                 255


          Ser Ala Thr Thr Thr Asn Ser Ile Pro Val Lys Gln Ala Ile Ala Gly
                     260                 265                 270


          Pro Gln Tyr Trp Asn Asn Glu Pro Asn Ser Thr Gly Ser Gln Ser Gly
                     275                 280                 285


          Lys Pro Arg Phe His Gly Asp Leu Asn Ser Ile Ser Ile Ala Ser Pro
                     290                 295                 300


          Asp Ile Asp Asp Thr Asn Ser Ser Phe Val Ser Leu Leu Ser His Gln
          305                 310                 315                 320
```

```
Leu Pro Gln Asn Ala Pro Phe Asn Thr Ser Thr Ile Val Gly Ser Leu
            325             330             335

Val Asn Gly Val Ser Arg Gln Gln Phe Ile Leu His Gly Met Asn Trp
            340             345             350

Ser Ser


<210>  8
<211>  353
<212>  PRT
<213>  Cucumis sativus

<400>  8

Met Met Glu Ser Thr Asp Ser Ser Ala Gly Pro Gln Gln Pro Asn Leu
1               5               10              15

Pro Pro Gly Phe Arg Phe His Pro Thr Asp Glu Glu Leu Val Val His
            20              25              30

Tyr Leu Lys Lys Lys Ala Asn Ser Ser Pro Leu Pro Val Ala Ile Ile
            35              40              45

Ala Glu Val Asp Leu Tyr Lys Phe Asp Pro Trp Glu Leu Pro Ala Lys
    50              55              60

Ala Thr Phe Gly Glu Gln Glu Trp Tyr Phe Phe Ser Pro Arg Glu Arg
65              70              75              80

Lys Tyr Pro Asn Gly Ala Arg Pro Asn Arg Ala Ala Thr Ser Gly Tyr
            85              90              95

Trp Lys Ala Thr Gly Thr Asp Lys Pro Val Leu Ala Ser Asp Gly Ser
            100             105             110

Asn Gln Lys Val Gly Val Lys Lys Ala Leu Val Phe Tyr Gly Gly Lys
            115             120             125

Pro Pro Lys Gly Ile Lys Thr Asn Trp Ile Met His Glu Tyr Arg Leu
            130             135             140

Ala Asp Asn Lys Pro Cys Ile Asn Lys Pro Pro Gly Tyr Asp Leu Ala
145             150             155             160

Asn Lys Lys Asn Ser Leu Lys Leu Asp Asp Trp Val Leu Cys Arg Ile
            165             170             175
```

```
Tyr Lys Lys Asn Asn Ser His Arg Pro Met Asp Gln Glu Arg Glu Asp
        180                 185                 190

Ser Met Glu Glu Met Ile Gly Ser Ile Pro His Ser Leu Arg Leu Asn
        195                 200                 205

Asp Gln Tyr Pro Lys Leu Gly Ile Asn Tyr Ser Thr Leu Leu Glu Asn
    210                 215                 220

Asp Gln Asn Leu Leu Gln Gly Ile Val Ala Asn Asn Asn Asn Asp Asn
225                 230                 235                 240

Asn Asn Asn Gly Ala Val Ser Asn Gly Thr Asn Ser Lys Arg Pro Ala
            245                 250                 255

Ser Leu Phe Trp Ser Asp Glu Asp Gln Asp His Ser Gly Ile Ser Ser
            260                 265                 270

Asn Lys Arg Leu His Phe Glu Asn Thr Thr Asp Gly Ala Ser Thr Ser
        275                 280                 285

Ile Thr Arg Thr His Ser Ser Ser His Asn Asn Leu Gln Asn Ser Thr
    290                 295                 300

Ser Ser Phe Thr Thr Leu Leu Thr Asn Leu Pro Gln Thr Pro Pro Pro
305                 310                 315                 320

Pro Leu His His His Ser Gly Ala His Ser Val Leu Ala Ser Ile Gly
            325                 330                 335

Asp Gly Leu Phe Arg Pro Ala Tyr Gln Ile Pro Gly Ala Asn Trp Tyr
            340                 345                 350

Ser
```

```
<210>  9
<211>  23
<212>  DNA
<213>  Artificial Sequence

<220>
<223>  Npt forward

<400>  9
atcaggatga tctggacgaa gag                                      23


<210>  10
<211>  22
<212>  DNA
```

<213> Artificial Sequence

<220>
<223> Npt Reverse

<400> 10
gataccgtaa agcacgagga ag                                                    22

<210> 11
<211> 49
<212> DNA
<213> Artificial Sequence

<220>
<223> RN forward

<400> 11
ggggacaagt ttgtacaaaa aagcaggcta tggagggcac caattcgtc                        49

<210> 12
<211> 50
<212> DNA
<213> Artificial Sequence

<220>
<223> RN Reverse

<400> 12
ggggaccact ttgtacaaga aagctgggtc tatgagttcc aattcaagct                       50

<210> 13
<211> 327
<212> PRT
<213> Helianthus annuus

<400> 13

Met Glu Ser Thr Asp Ser Ser Ser Gly Ser Lys Gln Pro Gln Leu Pro
1               5                   10                  15

Pro Gly Phe Arg Phe His Pro Thr Asp Glu Glu Leu Val Val His Tyr
            20                  25                  30

Leu Lys Lys Lys Ala Ala Ser Ala Pro Leu Pro Val Ala Ile Ile Ala
        35                  40                  45

Glu Val Asp Leu Tyr Lys Phe Asp Pro Trp Glu Leu Pro Ala Lys Ala
    50                  55                  60

Thr Phe Gly Glu Glu Glu Trp Tyr Phe Phe Ser Pro Arg Asp Arg Lys
65                  70                  75                  80

Tyr Pro Asn Gly Ala Arg Pro Asn Arg Ala Ala Thr Ser Gly Tyr Trp
                85                  90                  95

24

Lys Ala Thr Gly Thr Asp Lys Pro Val Leu Thr Ser Gly Gly Thr Gln
            100                 105                 110

Lys Val Gly Val Lys Lys Ala Leu Val Phe Tyr Gly Gly Lys Pro Pro
            115                 120                 125

Lys Gly Ser Lys Thr Asn Trp Ile Met His Glu Tyr Arg Leu Ala Asp
            130                 135                 140

Ser Lys Thr Ile Ser Lys Pro Pro Gly Cys Asn Pro Val Asn Lys Lys
145                 150                 155                 160

Ala Ser Leu Arg Leu Asp Asp Trp Val Leu Cys Arg Ile Tyr Lys Lys
                165                 170                 175

Asn Asn Ile Gln Arg Pro Val Asp Ser Asp Ser Asn Asp His Ala Met
            180                 185                 190

Thr Gly Met Leu Ala Ser Ile Pro Pro Ser Ile Ser Leu Arg Pro Thr
            195                 200                 205

Gly Phe Asn Thr Met Leu Glu Asn His Glu His Asn Val Val Phe Asp
            210                 215                 220

Ala Met Leu Thr Ser Asn Leu Asn Ser Thr Glu Asn Val Asn Ile Thr
225                 230                 235                 240

Thr Ser Asn Leu Leu Pro Val Lys Arg Ser Leu Pro Asn Phe Phe Trp
                245                 250                 255

Asn Asp Glu Gly His Thr Ser Asn Ser Pro Tyr Thr Lys Arg Phe Ile
            260                 265                 270

Ser Asp Ser Asn Ser Asp Gly Gly Val Leu Val Thr Arg Thr Asn Glu
            275                 280                 285

Glu Asn Asn Gly Gly Ser Ile Ala Asn Leu Leu Gly Gln Gln Gln Ala
            290                 295                 300

Leu Leu Gly Ser Val Gly Asp Thr Gly Val Tyr Arg Gln Gln Tyr Gln
305                 310                 315                 320

Leu Pro Ser Met Asn Trp Tyr
                325

<210> 14
<211> 314

```
<212>   PRT
<213>   Artificial Sequence

<220>
<223>   NAM Consensus

<400>   14

Met Glu Glu Asn Leu Pro Pro Gly Phe Arg Phe His Pro Thr Asp Glu
1               5                   10                  15


Glu Leu Ile Thr His Tyr Leu Cys Arg Lys Val Ser Asp Ile Gly Phe
            20                  25                  30


Thr Gly Lys Ala Val Val Asp Val Asp Leu Asn Lys Cys Glu Pro Trp
            35                  40                  45


Asp Leu Pro Ala Lys Ala Ser Met Gly Glu Lys Glu Trp Tyr Phe Phe
    50                  55                  60


Ser Gln Arg Asp Arg Lys Tyr Pro Thr Gly Leu Arg Thr Asn Arg Ala
65                  70                  75                  80


Thr Glu Ala Gly Tyr Trp Lys Thr Thr Gly Lys Asp Lys Glu Ile Tyr
                85                  90                  95


Arg Ser Gly Val Leu Val Gly Met Lys Lys Thr Leu Val Phe Tyr Lys
            100                 105                 110


Gly Arg Ala Pro Lys Gly Glu Lys Ser Asn Trp Val Met His Glu Tyr
            115                 120                 125


Arg Leu Glu Ser Lys Gln Pro Phe Asn Pro Thr Asn Lys Glu Glu Trp
    130                 135                 140


Val Val Cys Arg Val Phe Glu Lys Ser Thr Ala Ala Lys Lys Ala Gln
145                 150                 155                 160


Glu Gln Gln Pro Gln Ser Ser Gln Pro Ser Phe Gly Ser Pro Cys Asp
                165                 170                 175


Ala Asn Ser Ser Met Ala Asn Glu Phe Glu Asp Ile Asp Glu Leu Pro
            180                 185                 190


Asn Leu Asn Ser Asn Ser Ser Thr Ile Asp Tyr Asn Asn His Ile His
    195                 200                 205


Gln Tyr Ser Gln Arg Asn Val Tyr Ser Glu Asp Asn Thr Thr Ser Thr
    210                 215                 220
```

```
Ala Gly Leu Asn Met Asn Met Asn Met Ala Ser Thr Asn Leu Gln Ser
225             230             235             240


Trp Thr Thr Ser Leu Leu Gly Pro Pro Leu Ser Pro Ile Asn Ser Leu
            245             250             255


Leu Leu Lys Ala Phe Gln Ile Arg Asn Ser Tyr Ser Phe Pro Lys Glu
            260             265             270


Met Ile Pro Ser Phe Asn His Ser Ser Leu Gln Gln Gly Val Ser Asn
        275             280             285


Met Ile Gln Asn Ala Ser Ser Ser Ser Gln Val Gln Pro Gln Pro Gln
    290             295             300


Glu Glu Ala Phe Asn Met Asp Ser Ile Trp
305             310
```

## Description

### Field of the invention

The present invention is in the field of agronomy and of genetics where the overexpression of a specific NAC gene allows increased nitrogen use efficiency and/or increased biomass and/or increased root development.

### Background of the invention

High-input crop practices have increased crop productivity over the last decades but inefficient use of these inputs has led to considerable environmental harm. Nitrogen (N) fertilization is a leading factor that contributes to environmental degradation. Approximately 30-80% of N applied escapes to contaminate water and increase incidence of toxic algal blooms, contribute to greenhouse gas emissions, and increase incidence of disease vectors. As a result, there has been an increased interest in more sustainable agricultural systems. One major and indispensable approach is to develop crop varieties that can more efficiently utilize nitrogen. However, response to nitrogen is complex and involves multiple organs and processes. Because of the highly complex nature of nitrogen responses, breeding for increased nitrogen use efficiency as well as identification of factors that may regulate a suite of these processes have been difficult.

NAC transcription factors are one of the largest families of transcriptional regulators in plants. Genes in the NAC family have been shown to regulate a wide range of developmental processes. Members of the NAC gene family have also been shown to play important roles in the regulation of the transcriptional reprogramming associated with plant stress, biotic stress and abiotic stress, responses. However, the signaling mechanism is complex and a single NAC gene may respond to several stress factors. NAC1 has been suggested to control lateral root development in *Arabidopsis.*

WO 2017/0134439 discloses the effect of reduced expression of a NAC gene in maize so as to increase the drought resistance.

### Summary of the invention

A first aspect of the present invention is a DNA vector comprising a genetic cassette suitable for the expression in a host cell of a protein selected from the group consisting of SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13 or of a protein sharing at least 95% of identity with the proteins encoded by the said SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13.

A related aspect is a host cell, preferably a plant cell, and/or a transgenic plant comprising this DNA vector.

The transgenic plant is preferably selected from the group consisting of poplar (*Populus sp*), sugar beet (*Beta vulgaris*), soya (*Glycine max*), cotton, cucumber, sunflower and rapeseed (*Brassica napus*), more preferably being poplar (*Populus sp*) or sugar beet (*Beta vulgaris*).

Another aspect of the present invention is a method for increasing nitrogen use efficiency of a plant comprising to transform a plant cell with the vector as above and to regenerate a plant from this transformed cell.

Another aspect of the present invention is a method for identifying and/or obtaining (selecting) plants with increased nitrogen use efficiency, comprising the steps of:

- obtaining different genetic origins of a plant species,

- growing the said different genetic origins of the said plant species under low nitrogen conditions, and

- measuring in the leaves and/or in the roots of the plants the expression level of a protein selected from the group consisting of SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13, or of a protein sharing at least 95% of identity with the proteins encoded by the said SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13 or

the expression level of the messenger RNA encoding the said proteins, wherein higher expression of the said proteins or of the said messenger RNA is associated to higher nitrogen use efficiency.

Preferably, this method further comprises the step of selecting the plants with higher expression of the protein selected from the group consisting of SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13, or of the protein sharing at least 95% of identity with the protein encoded by the said SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13 or

with higher amount of a messenger RNA encoding this protein.

This method preferably further comprises the step of measuring the anthocyanin content in the leaves or in the roots of the plants.

In this method, preferably, the plants are selected from the group consisting of poplar (*Populus sp*), sugar beet (*Beta vulgaris*), soybean (*Glycine max*), rapeseed (Brassica napus), cotton, cucumber and sunflower more preferably being poplar (*Populus sp*) or sugar beet (*Beta vulgaris*).

Another aspect of the present invention is a plant or a plant part (e.g. a tissue, an explant or an isolated cell) or a seed obtainable by this method.

Still another aspect of the present invention is the use of this (transgenic) plant for a culture in soils with low nitrogen contents.

Another aspect of the present invention is a method for identifying and/or obtaining (selecting) a plant selected from the group consisting of poplar (*Populus sp*), sugar beet (*Beta vulgaris*), soybean (*Glycine max*), rapeseed (*Brassica napus*), cotton, cucumber and sunflower with increased root growth, comprising the steps of

- obtaining different genetic origins of a plant species,

- growing the said different genetic origins of the said plant species in a low-nitrogen soil, or in a soil with no nitrogen limitation, or both in a low-nitrogen soil and in a soil with no nitrogen limitation;

- measuring in the leaves and/or in the roots the expression level of

a protein selected from the group consisting of SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13, or of
a protein sharing at least 95% of identity with the proteins encoded by the said SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13
or of measuring the amount of a messenger RNA encoding the said proteins, and

- selecting the plants with higher expression level of the said protein or of the said messenger RNA.

Preferably, in these methods for identifying and/or obtaining (selecting) plants with increased nitrogen use efficiency or with increased root growth, the plants are grown in both a low-nitrogen soil and in a soil with no nitrogen limitation and wherein the selection step is based on both (i) the absolute expression level of the protein or of the messenger RNA when the plant is grown in low-nitrogen soil, and (ii) on the relative expression level of the protein or of the messenger RNA in a plant from one given genetic origin when grown in low-nitrogen soil by comparison with the culture of the plant from the same given genetic origin in a soil with no nitrogen limitation.

Another aspect of the present invention is a kit comprising an oligonucleotide sequence having between 20 and 100 contiguous nucleotides of a nucleotide sequence encoding SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13 or of a nucleotide sequence encoding a protein sharing at least 95% of identity with the proteins encoded by the said SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13, and instructions and/or reagents for use in marker-assisted selection of a plant selected from the group consisting of poplar (*Populus sp*), sugar beet (*Beta vulgaris*), soybean (*Glycine max*), rapeseed (*Brassica napus*), cotton, cucumber and sunflower.

**Detailed description of the invention**

The inventors have found plants having increased root growth under low nitrogen (LN) conditions. The inventors have identified the gene responsible of this advantageous feature (SEQ.ID.NOs 1-2), a member of the NAC transcription factor family (NAC 25). The over-activity of this NAC transcription factor (transgenic plants, but also a mutant, including a mutation in the promoter region) yields plants with yellow/red color, which usually reflects stress. Thus, generally, such plants are not selected for breeding purposes and breeders would rather select plants with a reduced expression of this NAC transcription factor. The present invention is nevertheless based on the opposite: plants with overexpression of this NAC transcription factor, despite having a more pronounced yellow/red color, have robust agronomic performances and allow improved yield.

In the WT (poplar) plants and under normal N conditions, this NAC transcription factor shows highest expression in leaves and roots. In WT plants, this NAC transcription factor (SEQ.ID.NOs 1-2) was significantly and rapidly (within hours) upregulated in the shoots but downregulated in the roots by LN.

Artificial down-regulation (i.e. by siRNA) of the gene led to reduced root growth, whereas its overexpression led to bigger roots compared to the original mutant.

Despite the multifaceted nature of the identified gene, a member of the NAC transcription factor, and also of the induced yellowing, its overexpression does not lead to negative pleiotropic changes that are often observed during overexpression of other transcription factors. Such pleiotropic phenotypes compromise performance under field conditions. In contrast, unexpectedly, this NAC transcription factor has been demonstrated by the inventors as able to orchestrate highly coordinated and balanced growth, developmental and physiological changes. Overexpression of this gene thus allows robust field performances.

A first aspect of the present invention is therefore a vector, preferably a DNA vector, comprising a genetic cassette suitable for the expression, or the over-expression, in an host cell of the NAC gene of the present invention, thus an overexpression of proteins such as of SEQ.ID.NO:2, or of SEQ.ID.NO:4 , or of a protein sharing at least 50%, preferably at least 75%, more preferably at least 80%, at least 90%, at least 95%, at least 99% of identity with SEQ.ID.NO:2 or SEQ.ID.NO:4, or being fully identical to SEQ.ID.NO:2 or SEQ.ID.NO:4.

Alternatively, the DNA vector comprises a genetic cassette suitable for the expression, or overexpression, in a host cell of a protein selected from the group consisting of SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13 or of a protein sharing at least 95% of identity with the proteins encoded by the said SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13.

In the present invention, the "percentage of identity" is advantageously measured using BLASTp algorithm and, unless otherwise specified, is preferably measured over the full-length proteins (both the Query and the Subject proteins).

Preferably, these vectors comprise a promoter that is not the endogenous promoter, advantageously a promoter allowing over-expression of the encoded gene.

Preferably, these encoded proteins sharing identity with SEQ.ID.NO:2, SEQ.ID.NO:4, SEQ.ID.NO:5, SEQ.ID.NO:6, SEQ SEQ.ID.NO:7, SEQ.ID.NO:8 and SEQ.ID.NO:13, but not sharing 100% of identity with these SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13, substantially share the activity of the proteins consisting of the said SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13; i.e. the activity is almost the same or even increased as compared with the activity of the wild-type protein, preferably, the activity is (substantially) not decreased.

Preferably, these encoded proteins, sharing at least 50% of identity with SEQ.ID.NO:2 or SEQ.ID.NO:4, is a functionally active (No Apical Meristem; NAM) transcription factor, more preferably sharing at least 50% of sequence identity with the N-terminal part (e.g. amino acids 5 to 150) of SEQ.ID.NO:14 (for instance aligning by BLASTp the target protein with amino acids 5-150 of SEQ.ID.NO:14).

These vectors are for use in increasing nitrogen use efficiency and/or for increasing root length of a plant.

A corresponding object is a host cell (prokaryotic, such as *E. coli* or eukaryotic, such as yeast or plant cell) comprising this DNA vector.

Another corresponding object is a transgenic plant comprising this DNA vector. Preferably the transgenic plant has been regenerated from a transgenic cell, meaning that all the cells are transgenic (comprise the transgenic trait of the present invention). However, chimeras (i.e. tissues with the transgenic cells of the present invention, and tissue without) are also encompassed if they share the phenotype of increased root length and/or increased nitrogen use efficiency.

Such transgenic plant is for use in increasing nitrogen use efficiency and/or for increasing root length of a plant.

Preferably, the plant or the transgenic plant cell of the present invention, for instance this host cell or transgenic plant, but also (non-transgenic) plants to be determined or obtained (or selected) for nitrogen use efficiency or for root length (see below) is belonging to the group of families consisting of *Amaranthaceae, Asteraceae, Betulaceae, Brassicaceae, Fabaceae, Fagaceae, Gossypieae, Juglandaceae, Oleaceae, Pinaceae, Poaceae, Rosaceae,* and *Salicaceae* and, more preferably, species selected from the group consisting of poplar (*Populus sp*), sugar beet (*Beta vulgaris*), soya (*Glycine max*), rapeseed (*Brassica napus*), cotton (*Gossypium hirsutum*), cucumber (*Cucumis sativum*), sunflower (*Helianthus annuus*), Maize (*Zea Mays*), birch (*Betula sp.*), oak (*Quecrus sp.*), beech (*Fagus sylvatica*), walnut (*Juglans sp.*), ash (*Fraxinus excelsior*), olive tree (*Olea europaea*), spruce (*Piceae abies*), larch (*Larix sp.*), pine (pinus *sp.*), fir (*Abies sp.*), Douglas fir (*Pseudotsuga menziesii*) still more preferably being poplar (*Populus sp*) or sugar beet (*Beta vulgaris*). The host cell can also be a prokaryotic cell, for instance to amplify the vector of the present invention.

Another related aspect of the present invention is a method for predicting nitrogen use efficiency of plants or for identifying and/or obtaining (selecting) plants with increased nitrogen use efficiency, comprising the steps of: obtaining different genetic origins of a plant species, of growing these genetic origins of a plant species under low nitrogen conditions, and measuring the expression level (e.g. in the roots) of a protein selected from the group consisting of SEQ.ID.NO:2 (poplar), SEQ.ID.NO:4 (sugar beet), SEQ.ID.NO:5 (soya), SEQ.ID.NO:6 (rapeseed), SEQ.ID.NO:7 (cotton), SEQ.ID.NO:8 (*cucumber*) and SEQ.ID.NO:13 (sunflower), or of a protein sharing at least 95% of identity with the proteins encoded by the said SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13 or the expression level of the messenger RNA encoding the said proteins, wherein higher expression of the said proteins or of the said messenger RNA in the roots and/or in the leaves is associated to higher nitrogen use efficiency.

The step of obtaining different genetic origins of a plant species can be performed by relying on a collection of plants, or by generating a collection of mutants, either using nonspecific mutagens, or by gene editing (e.g. CRISPR, TALEN,...).

The anthocyanin content can advantageously be further measured in the same plants, and higher anthocyanin content (or ratio) is a secondary indication of the overexpression of the NAC (NAC 25) genes described here above. Anthocyanin content can be displayed (measured) as the (molar) ratio anthocyanin/dihydroguercetin. Preferably, the step of measuring the expression of the above-mentioned proteins or mRNA (or anthocyanin) involves the comparison under normal nitrogen and low nitrogen conditions.

Preferably, this method further comprises the step of selecting the plants with higher (root) expression of the protein selected from the group consisting of SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13, or of the protein sharing at least 95% of identity with the protein encoded by the said SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13 or with higher amount (in roots) of a messenger RNA encoding the said protein and/or, optionally, with higher anthocyanin content.

Another related aspect of the present invention is a plant obtainable by the above methods.

The plant obtained or found by the above methods are advantageously used for a culture in soils with low nitrogen contents or in soils with low nitrogen supplementation.

Another related aspect of the present invention is a method for identifying and/or obtaining (selecting) a plant selected from the group consisting of poplar (*Populus sp*), sugar beet (*Beta vulgaris*), soya (*Glycine max*), rapeseed (*Brassica napus*), cotton (*Gossypium hirsutum*) and sunflower (*Helianthus annuus*) with increased root growth, comprising the steps of obtaining different genetic origins of this plant; growing different genetic origins of this plant in a low-nitrogen soil, or in a soil with no nitrogen limitation, or both in a low-nitrogen soil and in a soil with no nitrogen limitation; measuring in the roots the expression level of a protein selected from the group consisting of SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13, or of a protein sharing at least 95% of identity with the proteins encoded by the said SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13 or of measuring the amount of a messenger RNA encoding these proteins, and/or, optionally, of measuring the anthocyanin content; and of selecting the plants with higher expression level of this protein or of this messenger RNA and, optionally with higher anthocyanin content.

In the above methods for predicting nitrogen use efficiency or root growth of plants, advantageously, the plants from different genetic origins are grown in both a low-nitrogen soil and in a soil with no nitrogen limitation and the selection step is based on both (i) the absolute expression level of the protein or of the messenger RNA or of the anthocyanin content when the plant is grown in low-nitrogen soil, and (ii) on the relative root expression level of the protein or of the messenger RNA and/or, optionally, of the anthocyanin content in a plant from one given genetic origin when grown in a low-nitrogen soil by comparison with the culture of the plant from the same given genetic origin in a soil with no nitrogen limitation.

Still another aspect of the present invention is a kit comprising an oligonucleotide sequence having between 20 and 100 contiguous nucleotides from a nucleotide sequence encoding SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13 or from a nucleotide sequence encoding a protein sharing at least 95% of identity with the proteins encoded by the said SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13, and instructions and/or reagents for use in marker-assisted selection of a plant selected from the group consisting of poplar (*Populus sp*), sugar beet (*Beta vulgaris*), soya (*Glycine max*), rapeseed (*Brassica napus*), cotton (*Gossypium hirsutum*), cucumber (*Cucumis sativus*) and sunflower (*Helianthus annuus*).

This kit is especially useful for the detection of transgenic plants or of mutant plants (naturally-occurring mutation, mutation driven by mutagen agents, mutation due to edition of the gene) of the invention. Preferably, the nucleotide sequence present in the kit spans over a genetic event not naturally found in the wild-type plant (e.g. mutational event, exogenous DNA) .

Brief description of the figures

Figure 1. Identification and characterization of poplar NAC (25) gene (Red NAC; RN) via activation tagging.

Figure 2. RN promotes lateral root branching and delays shoot growth cessation under LN.

Examples

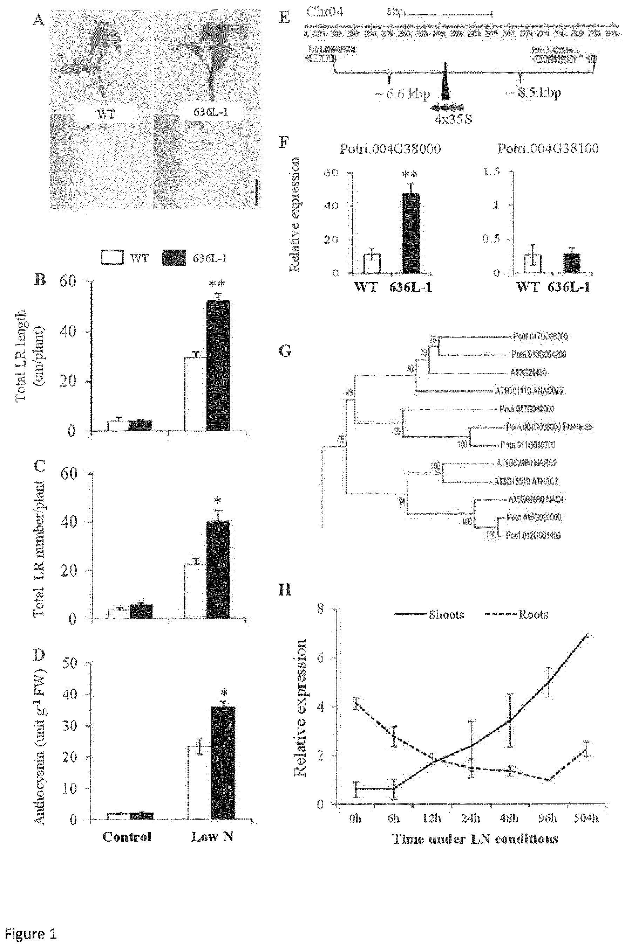

The inventors have firstly screened a population of poplar activation tagged lines for changes in root growth and morphology in relation to low nitrogen (LN). Under the *in vitro* conditions of the screen, line 636L-1 showed significantly increased root growth under LN conditions compared to WT plants, including root length of both primary and lateral roots as well as lateral root density (Fig. 1A-C). Interestingly, this line also displayed increased anthocyanin accumulation under LN compared to WT plants. Consistent with the increased anthocyanin accumulation, dihydroquercetin, the immediate precursor in the anthocyanin biosynthesis, was the most significantly changed among the metabolites with most dramatic changes in the mutant line (>2X vs. WT, P<0.05).

Table 1 Metabolites with significant change in the leaves of the mutant 636L-1 line.

| Metabolite | Control | | LN | |
|---|---|---|---|---|
| | Fold change | P-value | Fold change | P-value |
| dihydroquercetin | 2.67 | 0.022 | 0.41 | 0.366 |
| 17.16 179 salicyloyl conjugate | 2.65 | 0.046 | 0.15 | 0.002 |
| 18.52 488 171 219 coumaroyl conjugate | 2.61 | 0.003 | 0.23 | 0.017 |
| 17.48 488 236 219 171 coumaroyl conjugate | 2.58 | 0.007 | 0.23 | 0.017 |
| 16.7 456 441 complex phenolic | 2.44 | 0.034 | 0.58 | 0.158 |

Fold change values are relative to WT. P values are associated with statistical significance determined by Student t-test.

Interestingly, the LN treatment led to depletion of dihydroquercetin in the mutant line, suggesting an active processing of the precursor to anthocyanin under these conditions.

The tag in 636L-1 was positioned in the poplar genome (Fig. 1E). The expression of the closer gene (Potri.004G038000) flanking the tag insertion was highly elevated while the other (Potri.004G038100) was unaffected (Fig. 1F). The activation tagged Potri.004G038000 gene encodes a protein of unknown function showing homology to NAC transcription factors (Fig. 1G). Two, but not AtNAC1, *Arabidopsis* orthologs also of unknown function clustered in the same lineage (Fig. 1). SEQ.ID.NO:4-8 and 13 represent homologs or orthologs in sugar *beet (Beta vulgaris),* soya (*Glycine max*), rapeseed (*Brassica napus*), cotton (*Gossypium hirsutum*), cucumber (*Cucumis sativus*) and sunflower (*Helianthus annuus*).

Because of the distinct red phenotype under LN, the gene has been named RED NAC (RN) by the inventors. RN's highest expression in the WT plants and normal N conditions was in leaves and roots. RN was significantly and rapidly (within hours) upregulated in the shoots but downregulated in the roots by LN (Fig. 1H). To test if the RN mutation acts autonomously or involves generation of mobile signal the inventors performed reciprocal grafting and studied the distinct anthocyanin phenotype. The mutant scion on WT rootstock developed the typical high anthocyanin phenotype without the involvement of the mutant rootstock. Furthermore, the mutant top scion was able to increase the anthocyanin content in the WT rootstock. In contrast, the mutant rootstock with WT top scion was able to develop the typical high anthocyanin content but unable to increase the anthocyanin content of the WT top scion. This suggests that RN activation generates a mobile signal that can travel through the graft but only in a basipetal fashion.

The inventors then performed comparative transcriptomic analyses of the mutant vs WT plants in both leaves and roots under LN condition. Consistent with the phenotypic observations, the differentially-regulated genes in the 636L-1 leaves were mostly enriched in GO (Gene Ontology) categories associated with secondary metabolism, particularly from the phenylpropanoid/anthocyanin biosynthetic pathways. In roots, the differentially expressed genes in the mutant line were mostly enriched in GO processes linked to response to stimulus (GO:0050896), particularly ones associated with response to auxin (see also below for auxin measurements). Auxin-associated genes dominated the response to hormone stimulus (GO:0009725) with highest number of differentially regulated genes in the 636L-1 plants.

The inventors then transgenically overexpressed (RN-OE) and downregulated (RN-RNAi) the RN gene. The effect of the manipulations was validated using real time qRT-PCR. The inventors selected 3 lines in which RN was significantly up-(lines 35, 39 and 46) and down-regulated (15, 29 and 30) and subjected the transgenic plants to the same *in vitro* treatments as described above for the isolation of the mutant line. The RN-OE plants exhibited increased root growth and anthocyanin accumulation under LN. Generally, the strong overexpression under the 35S promoter produced much more pronounced phenotypes compared to the original mutant line. Conversely, RN downregulation led to decrease in both root growth and anthocyanin content under LN but only the most severely downregulated line (30-i) was significantly different in both traits. The lines with the largest RN downregulation (30-i) and upregulation (46-OE) and most significant phenotypic changes in both traits were selected for further analyses.

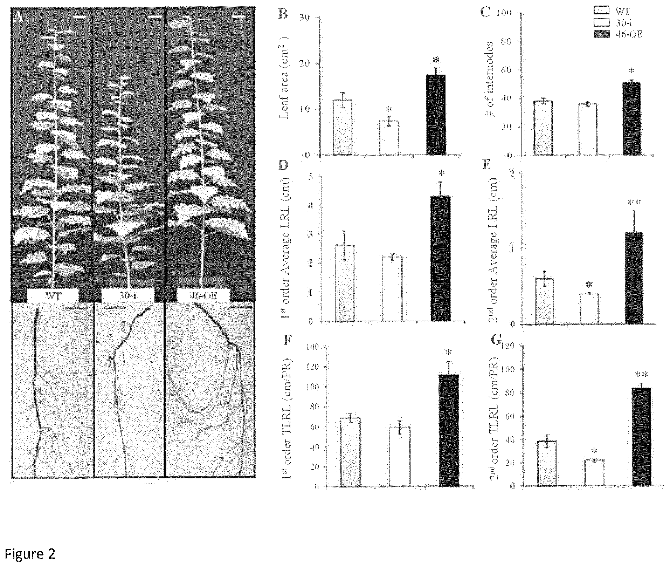

The inventors next grew the selected transgenic lines in greenhouse conditions and studied various aspects of their morphology and physiology under normal and LN fertilization regimes. Consistent with the *in vitro* observations, under LN in greenhouse conditions, the RN-modified transgenics showed highly significant changes in root architecture (Fig. 2, especially 2.A) and anthocyanin accumulation. Fig. 2 (B-G) Shoot and root characteristics of WT (grey), RN-RNAi (white) and RN-OE (black) plants grown under LN. '*' and '**' indicate significance at P<0.05 and P<0.01 as determined by Student t-test comparison to WT. Whole plant Scale = 2 cm, Root Scale = 1 cm. Most importantly RN overexpression produced a highly branched root system with longer fine roots while RN-RNAi roots were less branched and shorter. These trends were most pronounced in the second order branching (e.g., the finest and most metabolically active roots). Auxin is a major hormone that regulates root development. The inventors therefore measured free auxin in the three genotypes.

Indeed, the RN-OE plants displayed a significant increase in the free bioactive indole acetic acid (IAA) concentration in the roots and this was observed only under LN treatment. The aboveground growth and development was also significantly affected. Under the severe N deprivation regime, WT plants ceased shoot and leaf expansion and formed dormant bud (Fig. 2). In contrast, the growth cessation in response to N deprivation was significantly delayed in RN-OE transgenics (Fig. 2) . This resulted in a larger number of internodes and greater leaf area, particularly in the young expanding leaves (Leaf Plastochron Index=LPI 1-5). In contrast, the RN downregulation had the opposite effect which was most significant and pronounced with respect to leaf area (Fig. 2).

The inventors calculated the nitrogen use efficiency (NUE) with respect to dry lignocellulosic biomass production based on normal and LN supply. RN-OE plants displayed more than 3 fold higher NUE than WT. NUE of RN-RNAi plants was lower but not significantly different from WT.

To better understand the observed responses, the inventors measured the N content in the different genotypes, treatments and tissues. N levels in the RN-RNAi plants were the same as WT across all tissues and treatments. In a sharp contrast, the RN-OE plants displayed a highly significantly higher N foliar and bark content compared to WT plants. This response was much more pronounced under LN. Bark N content was also significantly higher but only under LN and at a much lower level than leaf content. Root N levels were unchanged in the three genotypes.

When grown under normal nitrogen fertilization regime, RN overexpression had a strong positive effect on a number of leaf and stem growth characteristics. Most importantly, RN-OE plants accumulated significantly more total dry leave and stem biomass. The latter constitutes the bulk of the lignocellulosic feedstock. Changes in growth often result in significant shifts in the cell wall content. The inventors therefore studied if the RN manipulations affected the cell wall composition. None of the three major cell wall components (e.g., cellulose, hemicellulose and lignin) was affected in either normal or LN conditions. Thus, the increased biomass growth of the RN-OE transgenics did not adversely impact the feedstock quality.

Materials and methods

*Plant materials and growth conditions*

A hybrid *Populus tremula* x *Populus alba* clone INRA 717-IB4 was used in all experiments and transgenic manipulations. Generation of the activation tagging population was performed using a binary vector pSKI074 and an Agrobacterium-mediated transformation. All putative transformants were PCR-verified for the presence of the neomycin phosphotransferase (NPT) selectable marker gene using the following primers: FwdP 5'-ATCAGGATGATCTGGACGAAGAG-3', (SEQ.ID.NO:9) and RevP 5'-GATACCGTAAAGCACGAGGAAG-3' (SEQ.ID.NO:10). Only NPT-positive lines were used in further experiments. Plants were *in vitro* propagated and maintained as known in the art. Plants for greenhouse experiments were first *in vitro* propagated and then acclimated to greenhouse conditions. All experiments were performed in 2L Belden square pots using 'Sunshine Growing Mix 3' soil. For control and LN treatments plants were supplied weekly with 50 ml of the same solutions used in the *in vitro* screening treatments (see below). Duration of experiments was as indicated in the figures' legends. Two-month-old plants of the WT and 636L-1 line were reciprocally grafted at approximately 50 cm height using the cleft method. The graft sites were allowed to heal for at least 1 month before treatments applied.

*In vitro screening for response to low nitrogen*

Stem cuttings of WT and transgenic lines were first synchronized for root development by cultivation for one week on 1/2 MS solid media containing 2 mg/l Indole Butyric Acid (IBA) in the dark. The cuttings were then directly transferred on filter paper bridges in glass tubes (2cm width, 25cm height, VWR) filled with 15 ml control (1/2 MS with 20mM $KNO_3$) or low N (LN) (1/2 MS with 0.05mM $KNO_3$) liquid solutions. The concentration of the $K^+$ in the LN treatment was accordingly adjusted with $K_2SO_4$. In both control and LN solutions, $KNO_3$ was the only N source. The explants were allowed to grow in the control and LN solutions for 40 days at 22°C and 16 h/8 h light/dark photoperiod. Post treatments, shoots and roots of each plant were scanned for measurements of different growth characteristics. For each treatment 4 clonal replicates per genotype were used.

*Biometric measurements*

Height, diameter and internode number were measured at the same time for the specific sets of experiments. Leaf area, root length and number were determined from digital pictures or scans using the ImageJ software. To determine dry weight, leaves, stems and roots were air-dried until their weight remained constant.

*Tag mapping and validation of gene activation*

Recovery of sequence flanking the insertion site of the activation tag was performed as known in the art. The isolated DNA fragment (Gene bank # KP176639) was positioned in the poplar genome using BLAST searches in the Phythozome v9.1 database and proximal genes to the insertion site identified. Expression of the flanking genes was studied using RT-PCR with gene specific primers and Ubiquitin gene as a loading control.

*Generation of constructs and transformation*

The open reading frame of RED NAC was amplified using the following primers:

B1-GGGGACAAGTTTGTACAAAAAAGCAGGCTATGGAGGGCACCAATTCGTC (SEQ.ID.NO:11) and B2-GGGGACCACTTTGTACAAGAAAGCTGGGTCTATGAGTTCCAATTCAAGCT (SEQ.ID.NO:12). The amplified product was then cloned into pDONR221 vector using the BP Gateway system (Invitrogen), sequence validated (Gene bank # KP176640) and transferred into the overexpression pK7WG2 and RNAi pK7GWIWG2(II) vectors using the LB Gateway system (Invitrogen).

*Sequence analyses*

Sequences of poplar and *Arabidopsis* NAC proteins were downloaded from the Phythozome v9.1 database. Sequence alignments were performed using the ClustalW method and phylogenetic analyses using MEGA4. The phylogenetic tree was constructed by the neighbor-joining method. The branch confidence was tested by bootstrap analyses of 1000 iterations.

*Microarray analyses*

Collection and analysis of microarray data were performed according to MIAME (Minimum Information About a Microarray Experiment) standards. Two independent biological replicates were used. Total RNA from roots and leaves was extracted and on-column DNase I-treated (Qiagen). Before labeling, RNA quality was assessed using the Agilent Bioanalyzer (Agilent Technologies) and 0.5 $\mu$g of total RNA was used to prepare biotinylated complementary RNA (cRNA). The poplar Affymetrix Poplar GeneChip was used (Affymetrix). The labeling, hybridization and imaging procedures were performed according to Affymetrix protocols. Raw microarray data was normalized using the robust multichip average with RMAExpress. Normalized expression data was analyzed using TM4:MeV software. Differentially expressed genes probe sets were identified using ANOVA (FDR q < 0.05, using Bioconductor QVALUE package), followed by posthoc contrast analyses using LIMMA with an FDR=0.01.

*Real time RT-PCR*

Total RNA was extracted as known in the art. Reverse transcription was performed on 1 $\mu$g of DNAase I-treated total RNA in a final reaction mixture of 20 $\mu$l using an M-MuLV (Moloney Murine Leukemia Virus) reverse transcriptase (Fermentas) following the manufacturer's protocol. Quantitative RT-PCR (qRT-PCR) was performed using the StepOnePlus Real Time System (Applied Biosystems) and the SYBR Green detection system. Each PCR reaction contained 1× Maxima SYBR Green qPCR master mix (Fermentas), 0.1 $\mu$M of each forward and reverse primer (Eurofins MWG Operon), 1 $\mu$l of 10× diluted cDNA solution and nuclease-free water. The final volume of each PCR reaction was 20 $\mu$l. The qRT-PCR cycling stages consisted of initial denaturation at 95°C for 10 min, followed by 40 cycles of 95°C for 15 s and 60°C for 1 min, and a final melting curve stage of 95°C for 15 s, 60°C for 1 min and 95°C for 15 s. Samples for qRT-PCR were run in three biological and two technical replicates. Relative gene expression was calculated. Ubiquitin (Ubq) gene expression was used as a loading control. All primers used in the gene expression analyses were designed from the *Populus* refseq mRNA sequences using the Primer-BLAST web resource at NCBI (National Center for Biotechnology Information).

*Cell wall analyses*

Wood samples were milled to 20-mesh using a Wiley mill. Approximately 4 mg of milled wood sample was measured, loaded into metal cups and arranged into an auto-sampler tray. Cell wall composition was studied by analyzing pyrolysis vapors produced using a commercially available molecular beam mass spectrometer (PyMBMS) designed specifically for biomass analysis as known in the art.

*Metabolic profiling*

Fast-frozen tissues were ground with liquid nitrogen in a chilled mortar (~25 mg fresh weight (FW) leaf and ~50 mg FW root) subsequently twice extracted with 2.5 mL 80% ethanol overnight and then combined prior to drying a 1.5 ml aliquot in a nitrogen stream. Sorbitol was added (to achieve ~22 ng/$\mu$L injected) before extraction as an internal standard to correct for differences in extraction efficiency, subsequent differences in derivatization efficiency and changes in sample volume during heating. Dried extracts were dissolved in 500 $\mu$L of silylation-grade acetonitrile followed by the addition of 500 $\mu$L N-methyl-N-trimethylsilyltrifluoroacetamide (MSTFA) with 1% trimethylchlorosilane (TMCS) (Thermo Scientific, Bellefonte, PA), and samples then heated for 1 h at 70 °C to generate trimethylsilyl (TMS) derivatives. After 2 days, 1-$\mu$L aliquots were injected into an Agilent Technologies Inc. (Santa Clara, CA) 5975C inert XL gas chromatograph-mass spectrometer, fitted with an Rtx-5MS with Integra-guard (5% diphenyl/95% dimethyl polysiloxane) 30 m x 250 $\mu$m x 0.25 $\mu$m film thickness capillary column. The standard quadrupole GC-MS was operated in the electron impact (70 eV) ionization mode, targeting 2.5 full-spectrum (50-650 Da) scans per second. Metabolite peaks were extracted using a key selected ion, characteristic m/z fragment, rather than the total ion chromatogram, to minimize integrating co-eluting metabolites. The extracted peaks of known metabolites were scaled back up to the total ion current using predetermined scaling factors. Peaks were quantified by area integration and the concentrations were normalized to the quantity of the internal standard (sorbitol) recovered, amount of sample extracted, derivatized, and injected. A large user-created database (>2100 spectra) of mass spectral electron impact ionization (EI) fragmentation patterns of TMS-derivatized compounds, as well as the Wiley Registry 8th Edition combined with NIST 05 mass spectral database, were used to identify the metabolites of interest to be quantified. Unidentified metabolites were denoted by their retention time as well as key mass-to-charge (m/z) ratios. Two biological replicate samples were analyzed per transgenic or control line and the metabolite data were presented as fold changed of the transgenic line versus the average of all of the control lines. The statistical significance of fold changes of the metabolite concentrations of transgenic lines versus control plants were determined by Student's t-tests.

*Anthocyanin measurements*

Anthocyanin content of leaves was determined as known in the art. All measurements were performed using Lambda 2 UV/VIS Spectrometer (Perkin Elmer).

*Auxin measurements*

For auxin analyses, leaf and root samples were harvested, frozen in liquid nitrogen. Root samples were then triple-ground in liquid nitrogen, and 20 mg was subjected to a secondary grinding, also in liquid nitrogen. Ground root samples were extracted with sodium phosphate buffer, as described previously, with the following modifications: all procedures were performed at 4°C under a yellow safe light, and 25ng [$^2$H$_5$] IAA (OlChemIm, Ltd., Olomouc, Czech Rebuplic) was added as an internal standard. Extracted auxin was then concentrated via solid-phase extraction (SPE), using 30 mg Waters HLB Columns (Waters, Corp., Milford, MA). Finally, SPE-purified samples were dried under nitrogen gas, re-dissolved in 1.00 mL LC-MS/MS grade methanol, passed through a 0.2 micron nylon filter (Fisherbrand, Fisher Scientific International, Hampton, NH), and subjected to LC-MS/MS analysis.

LC-MS/MS analyses were performed on an Agilent 6460 QQQ Triple-quadrupole mass spectrometer in multiple reaction monitoring (MRM) mode, linked to an Agilent 1260 Infinity HPLC (Agilent Technologies, Santa Clara, CA). Auxin and auxin metabolites were separated using an Agilent Poroshell 120 EC-C18 column (3.5 x 50 mm, 2.7 micron, Agilent Technologies, Santa Clara, CA). Separations used a two-buffer system (A: 0.1% acetic acid, 5% methanol, 94.9% 18.3 megaOhm water; B: 0.1% acetic acid in methanol) and the following gradient conditions: 2min 0 to 10% B, 4min 10 to 60% B, 5min 60 to 100% B, and 3 minute hold at 100% B. Mass spectrometry analyses were performed using electrospray ionization (ESI), with the following settings: gas temperature, 250 °C; gas flow, 10L/min; nebulizer pressure, 60psi; sheath gas flow, 12 L/min; sheath gas temperature, 400°C; capillary voltage, 2250V; Nozzle voltage, 500V. Mass peak for auxin was confirmed and quantified via comparison to standard curves generated using authentic standard, Sigma-Aldrich, St. Louis, MO [indole-3-acetic acid]. Specific mass transition monitored for indole-3-acetic acid (IAA) was: RT=9.6 min., 176→130 and 176→77.

*Measurement of N and NUE*

To quantify N, wood, bark, and leaves tissues were dried, milled to fine powder, 5mg was weighed into tin capsules and analyzed by an element analyzer (Fisons NA1500 Elemental Analyzer). The instrument was calibrated with atropine, and NIST 1547 (peach leaves) were run every 12 samples to check for instrument stability. The average of NIST 1547 was 2.93 %N with a standard deviation of 0.05 (n=14). Nitrogen use efficiency (NUE) was calculated using the following formula:

```
Dry stem biomass (control treatment) - Dry stem biomass (low

nitrogen treatment)/Total applied nitrogen (control treatment)

- Total applied nitrogen (low nitrogen treatment
```

**Claims**

1.  A DNA vector comprising a genetic cassette suitable for the expression in a host cell of a protein selected from the group consisting of SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13 or of a protein sharing at least 95% of identity with the proteins encoded by the said SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13.

2.  A host cell, preferably a plant cell, comprising the DNA vector of claim 1.

3.  A transgenic plant comprising the DNA vector of claim 1.

4.  The transgenic plant of claim 3 being selected from the group consisting of poplar (*Populus sp*), sugar beet (*Beta vulgaris*), soya (*Glycine max*), cotton, cucumber, sunflower and rapeseed (*Brassica napus*), more preferably being poplar (*Populus sp*) or sugar beet (*Beta vulgaris*).

5.  A method for increasing nitrogen use efficiency of a plant comprising to transform a plant cell with the vector of claim 1 and to regenerate a plant from the said transformed cell.

6.  A method for identifying plants with increased nitrogen use efficiency, comprising the steps of:

    - obtaining different genetic origins of a plant species,
    - growing the said different genetic origins of the said plant species under low nitrogen conditions, and
    - measuring in the leaves and/or in the roots of the plants the expression level of a protein selected from the group consisting of SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13, or of a protein sharing at least 95% of identity with the proteins encoded by the said SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13 or

    the expression level of the messenger RNA encoding the said proteins, wherein higher expression of the said proteins or of the said messenger RNA is associated to higher nitrogen use efficiency.

7.  The method of claim 6 further comprising the step of selecting the plants with higher expression of the protein selected from the group consisting of SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13, or of the protein sharing at least 95% of identity with the protein encoded by the said SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13 or

with higher amount of a messenger RNA encoding the said protein.

8. The method of claims 6 or 7 further comprising the step of measuring the anthocyanin content in the leaves or in the roots of the plants.

9. The method according to any of the preceding claims 6 to 8, wherein the plants are selected from the group consisting of poplar (*Populus sp*), sugar beet (*Beta vulgaris*), soybean (*Glycine max*), rapeseed (*Brassica napus*), cotton, cucumber and sunflower preferably being poplar (*Populus sp*) or sugar beet (*Beta vulgaris*).

10. A plant, a plant part or a seed obtainable by the method of claims 6 to 9.

11. Use of the plant according to any one of the preceding claims 3 to 4 or of the plant or the seed of claim 10 for a culture in soils with low nitrogen contents.

12. A method for identifying a plant selected from the group consisting of poplar (*Populus sp*), sugar beet (*Beta vulgaris*), soybean (*Glycine max*), rapeseed (*Brassica napus*), cotton, cucumber and sunflower with increased root growth, comprising the steps of

- obtaining different genetic origins of a plant species,
- growing the said different genetic origins of the said plant species in a low-nitrogen soil, or in a soil with no nitrogen limitation, or both in a low-nitrogen soil and in a soil with no nitrogen limitation;
- measuring in the leaves and/or in the roots the expression level of

a protein selected from the group consisting of SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13, or of a protein sharing at least 95% of identity with the proteins encoded by the said SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13
or of measuring the amount of a messenger RNA encoding the said proteins, and

- selecting the plants with higher expression level of the said protein or of the said messenger RNA.

13. The method according to any one of the preceding claims 6 to 9 or 12, wherein the plants are grown in both a low-nitrogen soil and in a soil with no nitrogen limitation and wherein the selection step is based on both (i) the absolute expression level of the protein or of the messenger RNA when the plant is grown in low-nitrogen soil, and (ii) on the relative expression level of the protein or of the messenger RNA in a plant from one given genetic origin when grown in low-nitrogen soil by comparison with the culture of the plant from the same given genetic origin in a soil with no nitrogen limitation.

14. A kit comprising
an oligonucleotide sequence having between 20 and 100 contiguous nucleotides of a nucleotide sequence encoding SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13 or of a nucleotide sequence encoding a protein sharing at least 95% of identity with the proteins encoded by the said SEQ.ID.NO:2, SEQ.ID.NO:4, SEQ.ID.NO:5, SEQ.ID.NO:6, SEQ.ID.NO:7, SEQ.ID.NO:8 and SEQ.ID.NO:13, and instructions and/or reagents for use in marker-assisted selection of a plant selected from the group consisting of poplar (*Populus sp*), sugar beet (*Beta vulgaris*), soybean (*Glycine max*), rapeseed (*Brassica napus*), cotton, *cucumber* and sunflower.

Figure 1

Figure 2

Europäisches
Patentamt

European
Patent Office

Office européen
des brevets

## EUROPEAN SEARCH REPORT

Application Number

EP 17 19 7076

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE UniProt [Online]<br><br>24 March 2009 (2009-03-24),<br>"SubName: Full=No apical meristem family protein {ECO:0000313¦EMBL:EEE86188.1};",<br>XP002778352,<br>retrieved from EBI accession no.<br>UNIPROT:B9H255<br>Database accession no. B9H255<br>* the whole document *<br>& TUSKAN G A ET AL:  "The genome of black cottonwood, Populus trichocarpa (Torr. & Gray)",<br>SCIENCE, AMERICAN ASSOCIATION FOR THE ADVANCEMENT OF SCIENCE,<br>vol. 313, no. 5793,<br>1 September 2006 (2006-09-01), pages 1596-1604, XP002591579,<br>ISSN: 0036-8075, DOI:<br>10.1126/SCIENCE.1128691 | 1-4,10,14 | INV.<br>C07K14/415<br>C12N15/82 |
| X | DATABASE Protein [Online]<br><br>29 November 2016 (2016-11-29),<br>"PREDICTED: NAC transcription factor 25 [Beta vulgaris subsp. vulgaris]",<br>XP002778353,<br>retrieved from NCBI<br>Database accession no. XP_010686575<br>* the whole document * | 1-4,10,14 | TECHNICAL FIELDS SEARCHED (IPC)<br><br>C07K<br>C12N |
| X | WO 2006/004955 A2 (CERES INC [US]; ALEXANDROV NICKOLAI [US]; BROVER VYACHESLAV [US]; MASC)<br>12 January 2006 (2006-01-12)<br>* claims 1, 4, 7, 9, 15, 17, 19; sequence 1251 * | 1-4,10,14 | |

-/--

The present search report has been drawn up for all claims

| Place of search | Date of completion of the search | Examiner |
|---|---|---|
| Munich | 23 February 2018 | Krüger, Julia |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 1 of 3

Europäisches
Patentamt

European
Patent Office

Office européen
des brevets

## EUROPEAN SEARCH REPORT

Application Number

EP 17 19 7076

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE UniProt [Online]<br><br>29 October 2014 (2014-10-29),<br>"SubName: Full=BnaA09g13880D protein {ECO:0000313¦EMBL:CDY12886.1};",<br>XP002778354,<br>retrieved from EBI accession no.<br>UNIPROT:A0A078FI47<br>Database accession no. A0A078FI47<br>* sequence *<br>& CHALHOUB BOULOS ET AL:  "Early allopolyploid evolution in the post-Neolithic Brassica napus oilseed genome",<br>SCIENCE (WASHINGTON D C),<br>vol. 345, no. 6199, August 2014 (2014-08), pages 950-953,<br>ISSN: 0036-8075(print)<br>----- | 1-4,10,14 | |
| X | DATABASE UniProt [Online]<br><br>10 May 2017 (2017-05-10),<br>"SubName: Full=NAC transcription factor 25-like {ECO:0000313¦RefSeq:XP_016755539.1};",<br>XP002778389,<br>retrieved from EBI accession no.<br>UNIPROT:A0A1U8PZ36<br>Database accession no. A0A1U8PZ36<br>* sequence *<br>& LI FUGUANG ET AL:  "Genome sequence of cultivated Upland cotton (Gossypium hirsutum TM-1) provides insights into genome evolution",<br>NATURE BIOTECHNOLOGY,<br>vol. 33, no. 5, May 2015 (2015-05), page 524,<br>ISSN: 1087-0156(print)<br>-----<br>-/-- | 1-4,10,14 | TECHNICAL FIELDS SEARCHED (IPC) |

The present search report has been drawn up for all claims

| Place of search | Date of completion of the search | Examiner |
|---|---|---|
| Munich | 23 February 2018 | Krüger, Julia |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 2 of 3

Europäisches
Patentamt

European
Patent Office

Office européen
des brevets

**EUROPEAN SEARCH REPORT**

Application Number

EP 17 19 7076

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE UniProt [Online]<br><br>7 January 2015 (2015-01-07),<br>"SubName: Full=Uncharacterized protein {ECO:0000313¦EMBL:KGN56220.1};",<br>XP002778390,<br>retrieved from EBI accession no.<br>UNIPROT:A0A0A0L2L3<br>Database accession no. A0A0A0L2L3<br>* sequence *<br>& HUANG SANWEN ET AL: "The genome of the cucumber, Cucumis sativus L.",<br>NATURE GENETICS,<br>vol. 41, no. 12, December 2009 (2009-12), page 1275,<br>ISSN: 1061-4036<br>----- | 1-4,10, 14 | |
| X | WO 2008/034648 A1 (METANOMICS GMBH [DE]; PUZIO PIOTR [DE]; BLAU ASTRID [DE]; WALK TILMANN) 27 March 2008 (2008-03-27)<br>* claims 13,24,27; sequence 2296 *<br>----- | 1-4,10, 14 | |
| A | GUOHUA XU ET AL: "Plant Nitrogen Assimilation and Use Efficiency",<br>ANNUAL REVIEW OF PLANT BIOLOGY,<br>vol. 63, no. 1, 2 June 2012 (2012-06-02), pages 153-182, XP055114006,<br>ISSN: 1543-5008, DOI:<br>10.1146/annurev-arplant-042811-105532<br>* figure 1; table 1 *<br>----- | 1-14 | TECHNICAL FIELDS SEARCHED (IPC) |

The present search report has been drawn up for all claims

| Place of search | Date of completion of the search | Examiner |
|---|---|---|
| Munich | 23 February 2018 | Krüger, Julia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 17 19 7076

This annex lists the patent family members relating to the patent documents cited in  the above-mentioned European search report.
The members are as contained in the European Patent Office EDP file on
The European Patent Office is in no way liable for these particulars which are merely given for the purpose of information.

23-02-2018

| Patent document cited in search report | | Publication date | Patent family member(s) | | Publication date |
|---|---|---|---|---|---|
| WO 2006004955 | A2 | 12-01-2006 | US 2006057724 A1 | | 16-03-2006 |
| | | | US 2009133156 A1 | | 21-05-2009 |
| | | | US 2013139278 A1 | | 30-05-2013 |
| | | | WO 2006004955 A2 | | 12-01-2006 |
| WO 2008034648 | A1 | 27-03-2008 | AU 2007299219 A1 | | 27-03-2008 |
| | | | CA 2644273 A1 | | 27-03-2008 |
| | | | EP 2090662 A2 | | 19-08-2009 |
| | | | WO 2008034648 A1 | | 27-03-2008 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 20170134439 A